(19) Europäisches Patentamt
European Patent Office
Office européen des brevets

(11) EP 2 080 510 B1

(12) **EUROPEAN PATENT SPECIFICATION**

(45) Date of publication and mention of the grant of the patent:
**20.08.2014 Bulletin 2014/34**

(21) Application number: **07830480.5**

(22) Date of filing: **24.10.2007**

(51) Int Cl.:
***A61K 9/127*** *(2006.01)*

(86) International application number:
**PCT/JP2007/070746**

(87) International publication number:
**WO 2008/050807 (02.05.2008 Gazette 2008/18)**

# (54) METHOD FOR PRODUCTION OF LIPOSOME PREPARATION

VERFAHREN ZUR HERSTELLUNG EINER LIPOSOM-ZUBEREITUNG

PROCEDE DE PRODUCTION D'UNE PREPARATION DE LIPOSOME

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **25.10.2006 JP 2006290298**

(43) Date of publication of application:
**22.07.2009 Bulletin 2009/30**

(73) Proprietor: **Terumo Kabushiki Kaisha**
**Tokyo 151-0072 (JP)**

(72) Inventors:
- **YOSHINO, Keisuke**
**Ashigarakami-gun**
**Kanagawa 259-0151 (JP)**
- **YAMASHITA, Keiko**
**Ashigarakami-gun**
**Kanagawa 259-0151 (JP)**
- **OGATA, Yoshitaka**
**Ashigarakami-gun**
**Kanagawa 259-0151 (JP)**

(74) Representative: **TBK**
**Bavariaring 4-6**
**80336 München (DE)**

(56) References cited:

| | |
|---|---|
| **EP-A1- 1 190 705** | **EP-A2- 0 800 822** |
| **WO-A1-01/00173** | **WO-A1-86/01102** |
| **JP-A- 05 504 150** | **JP-A- 07 165 560** |
| **US-A- 5 393 530** | **US-A- 5 939 096** |
| **US-A1- 2004 071 768** | **US-B1- 6 355 268** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 080 510 B1

Printed by Jouve, 75001 PARIS (FR)

## Description

### Technical Field

This present invention relates to a method for the production of a liposome preparation with a desired drug loaded in liposomes.

### Background Art

In recent years, active research is underway on drug delivery systems (DDS) for the purpose of achieving extended release (sustained release) of drug, prolongation of the life of drug the in vivo half-life of which is short, promotion of absorption of drug at varied lesion sites, or delivery of drug to a target tissue or cells only as intended. DDS technologies include sustained release technologies and targeting technologies. The former technologies allow gradual release of a drug from its preparation so that the blood level of the drug can be maintained constant over a long term to sustain its effects. The latter technologies selectively and efficiently deliver a drug to an inflamed site or cancer cells as a target. As drug carriers for achieving such DDS technologies, it is contemplated to use sealed vesicles such as liposomes, emulsions, lipid microspheres or nanoparticles. For their practical application, however, there are various problems to be overcome.

Upon using liposomes in DDS, loading efficiency, storage stability and the like of a drug are required. As a method for achieving a high drug loading efficiency, there is a remote loading method that forms liposomes beforehand and loads a drug into the liposomes while making use of an ion gradient or pH gradient which occurs between an internal water phase and an external water phase of each liposome (see Patent Document 1, etc.).

The incorporation of a drug into liposomes by such a remote loading method is conducted under heat at or above the phase transition point of phospholipids that make up membranes of the liposomes. It is a common practice to heat a mixture of a suspension of liposomes, into which the incorporation of a drug is intended, and a solution of the drug at approx. 65°C for 30 minutes (see, Patent Document 2, etc.).

Patent Document 1: Japanese Patent No. 2659136
Patent Document 2: PCT Patent Publication No. WO2005/092388 Pamphlet

Further, US 6355268 B1 discloses liposome entrapped topoisomerase inhibitors. In an Example, the liposomes were mixed with a selected drug solution and rapidly warmed to 65°C using a pre-equilibrated jacketed vessel containing water.

US 5939096 A discloses a liposome drug-loading method. The method employs a proton shuttle mechanism involving the salt of a weak acid to generate a higher inside/lower outside pH gradient.

EP 0800822 A2 discloses a method for preparing a closed vesicle by a rehydration of a closed vesicle using a rehydration solution.

EP 1190705 A1 discloses a method of inhibiting the leakage of a drug encapsulated in liposomes.

US 2004/071768 A1 discloses liposome-encapsulated vinca alkaloids and methods of treating a mammal using such compositions.

WO 86/01102 A1 discloses methods for encapsulating ionizable antineoplastic agents in liposomes using transmembrane potentials.

US 5393530 A discloses a method for making liposomes. Liposome vesicles are prepared containing water or very dilute solutions encapsulated therein. These "empty" liposomes are suspended in a carrier liquid containing, dispersed therein, substances of interest to be loaded into the vesicles and incubated for a period of time at temperatures above the lipids transition temperature, whereby loading by transmembrane permeation occurs.

### Disclosure of Invention

### Technical Problem

Concerning the production of a liposome preparation by a method that includes a drug incorporation step by such a remote loading method as described above, the heating in the drug incorporation step becomes a problem whenever an attempt is made to practice this method on an industrial level. Described specifically, in the remote loading method, the drug is generally loaded into liposomes by mixing a suspension of the liposomes, in each of which an ion gradient or pH gradient has been caused to occur between its internal water phase and its external water phase, and a solution of the drug at a temperature equal to or higher than the phase transition point of the lipid membranes of the liposomes. In the drug incorporation step as described above, the liposome suspension and the drug solution are separately heated beforehand to a temperature equal to or higher than the phase transition point of the lipid membranes of the liposomes and are then mixed together, and after the mixing, the mixture is also maintained in a heated state for a predetermined time until the loading is completed. However, the greater the production scale, the longer the time required beforehand for the temperature rise prior to the mixing. Moreover, at a large production scale, long-time heating is also needed at the time of the mixing to evenly heat the entirety, leading to a concern that the physiochemical stability of the liposomes and the drug under loading may be affected. In particular, liposomes with a pH gradient developed therein, which are before the loading of a drug therein, are known to be susceptible to the formation of lipid degradation products (lipidolysis) compared with the same liposomes after the loading of the drug therein. From this standpoint, the step that heats the liposome suspension beforehand prior to the loading of the drug is not preferred. When the production scale is large, it is necessary to mix the drug little by little into the liposome suspension in order to load the drug evenly into the respective liposome particles, and therefore, a long time is required for the mixing. In other words, the liposomes in the unloaded form before their contact with the drug remain to exist over a long time during the mixing. This is not preferred either. Further, after the completion of the drug incorporation, the thermal stability of the liposomes is improved. Nonetheless, any unnecessary thermal history should be avoided, and therefore, the temperature needs to be promptly lowered. However, a large production scale also needs time for a temperature down.

On the other hand, the production process of liposomes proceeds through steps in multiple stages as mentioned above, and therefore, the time needed for the production is long. In small-scale production, for example, at a laboratory level, the time aspect is not very important because the time to be spent for a temperature rise and a temperature down is short. When a medium- or large-scale production method is assumed, however, the time to be spent for a temperature rise and a temperature down cannot be ignorable, and the shortening of production time is also essential from the standpoint of production cost. In the medium- or large-scale production method, it is also necessary to take into consideration the equipment for the production process. To maintain the temperature, for example, there is a need to produce by using a tank equipped with a jacket heating device. Such a tank requires a high equipment investment cost. An advance investigation is therefore needed to minimize such steps under a detailed design. The production of liposomes generally includes the step that is performed at a temperature equal to or higher than the phase transition point of lipid membranes, and therefore, requires equipment for raising the temperature and maintaining the temperature. This production, however, also includes a step for which heating at the phase transition point or higher is not desired. The overall process, therefore, needs a step to effect a temperature reduction after effecting a temperature rise. This is one of reasons for the lengthy production process.

Further, upon industrial practice of the remote loading method, the uniformity in the drug incorporation step exists as a matter of concern.

Technical Solution

An object of the present invention is to develop a simple and easy production method, which in a medium- or large-scale production method, makes it possible to significantly shorten the production time of liposomes to achieve a substantial cost cut-down, to produce a preparation with a uniform amount of incorporated drug from the standpoint of a quality aspect, and further to suppress lipid degradation by a substantial reduction in thermal exposure time.

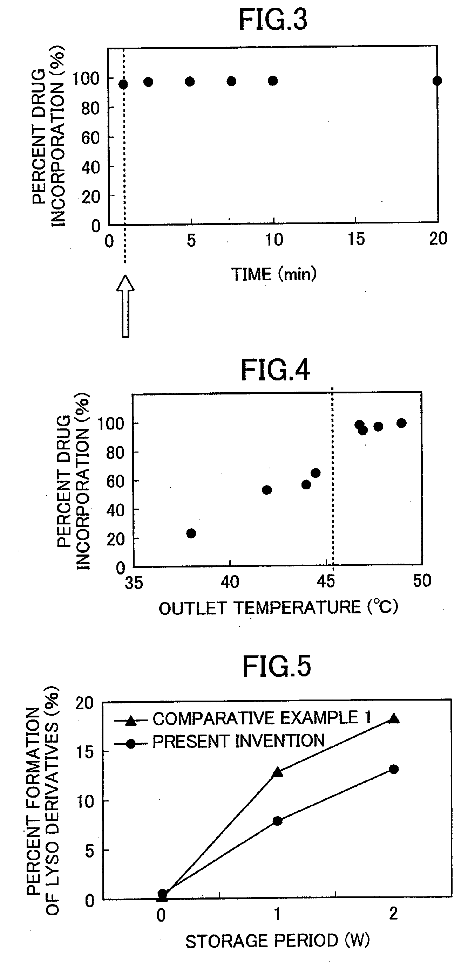

Especially, as a result of an investigation by the present inventors especially about the drug incorporation step by a remote loading method, it was found that, as will be described as Examples subsequently herein, the drug incorporation step is completed in one minute in a small-scale reaction when heated to a temperature equal to or higher than the phase transition point of lipid membranes (see FIG. 3). However, this fact is available in small-scale reactions. When the production scale became greater, a time of one minute or longer was needed in the step in which a liposome suspension and a drug solution are mixed (see Comparative Examples to be described subsequently herein). It was, hence, appreciated that in such a large-scale reaction, there is indicated a concern about irregularity in the amount of incorporated drug among the liposomes in a preparation. Described specifically, even when a drug is added little by little to a liposome suspension as mentioned above, the drug exist in an excess amount for some of the liposomes in the liposome suspension, with which the drug solution come into contact at a beginning, and is sufficiently incorporated (loaded) in the some liposome. Gradually, however, the drug decreases in amount and disperses in the liposome suspension. Therefore, the amount of the drug to be incorporated in the liposomes in the liposome suspension, with which the drug solution then newly come into contact, gradually decreases. As an alternative, the charged drug is consumed (incorporated) in its entirety by some of the liposomes before it is distributed to all the liposome particles, and is not incorporated in the remaining liposomes or, even when incorporated, liposome particles with the drug incorporated in smaller amounts occur.

If the drug is charged in a large excess, the drug is loaded (incorporated) as much an amount as possible in all the liposome particles so that a uniform preparation may be obtained. However, this approach results in a great deal of unloaded drug, and therefore, is not practical at all. The exhibition of activities by a drug loaded in liposomes stems from the disposition of the liposomes themselves, and irregularity among preparations, such as differences in the amount of introduced drug, is likely to considerably affect their drug activity. It is, therefore, necessary to choose a production process that can assure uniformity.

As described above, with the processes practiced at present, the production of a uniform and stable preparation may be feasible in some instances when the production is small-scale production at a laboratory level. As the production scale increases to medium- or large-scale production, a need arises for a process that requires a considerable time. Due to an increase in the thermal exposure time, the physiochemical stability of the preparation is also concerned. Further, in view of the drug incorporation time in the drug incorporation step, the irregularity in the amount of incorporated drug among preparations is also concerned. From the standpoints of these three matters, specifically the cost aspect, the physiochemical stability of a preparation and the quality aspect, there is a concern about the application of the drug incorporation step to medium- or large-scale production for its practical use. It is, however, the current circumstance that there is not any method which has overcome these concerns and the drug incorporation step has to be practiced under insufficient production conditions.

Based on the above findings, the present invention is provided as will be described hereinafter.

(1) A method for producing a liposome preparation by using a remote loading method, which includes a drug incorporation step that heats a mixture of a preliminarily prepared suspension of liposomes and a drug by rapid heating means to a temperature from not lower than a phase transition point of membranes of the liposomes to not higher than 80°C to incorporate the drug into the liposomes.

The method may further include a step that mixes the suspension of the liposomes and the drug at a temperature not higher than the phase transition point of the membranes of the liposomes prior to the drug incorporation step.

The drug incorporation step in the present invention is based on the remote loading method. Specifically,

(2) The method as described above in (1), wherein the drug is incorporated into the liposomes by an ion gradient method.

According to the invention, the rapid heating means is a heat exchanger interposed in a feed pipe.

The heat exchanger may preferably be a capillary heat exchanger, and the diameters (inner diameters) of the capillaries are generally from 0.5 to 15 mm.

(3) The method as described above in (1), wherein a heat-exchanger residence time in the heat exchanger is from 3 to 120 seconds.

(4) The method as described above in any one of (1) to (3), which includes performing an elimination step for any unloaded drug after the drug incorporation step. Advantageous Effects

Effects of the Invention

According to the present invention, the production method according to the present invention which has taken into consideration an application to medium- or large-scale production for its practical use is a method that can substantially shorten the production time while maintaining the conventional loading amount and loading efficiency, and can be fully satisfactory from the standpoint of production cost. Further, the production method according to the present invention is a method that induces no irregularity in the amount of incorporated drug among preparations, because the liposome suspension and the drug solution are mixed together into a uniform mixture at a temperature at which no drug incorporation takes place and by using a heat exchanger, the uniform mixture is then heated in a short time to the phase transition point or higher and the drug can hence be incorporated during the feeding of the mixture. The method according to the present invention is, therefore, a method that can be fully satisfactory from the standpoint of quality aspect. Furthermore, the degradation of lipids can also be significantly suppressed owing to the substantial shortening of the thermal exposure time. For the foregoing, the production method according to the present invention can be fully satisfactory from the standpoint of production time, the standpoint of quality aspect such as uniformity in the amount of incorporated drug, and the standpoint of the stability of the preparation.

Brief Description of Drawings

[FIG. 1] is a schematic illustration diagram of one embodiment of the drug incorporation step in the method according to the present invention.

[FIG. 2] is a diagram showing drug incorporation rates at respective temperatures.

[FIG. 3] is a diagram showing the percent incorporation of a drug versus drug introduction time at 50°C in an investigation on the percent incorporation of the drug.

[FIG. 4] is a diagram illustrating a relationship of the percent incorporation of a drug to outlet temperature in a drug incorporation step making use of a heat exchanger.

[FIG. 5] is a diagram illustrating the storage stability (percent formation of lyso derivatives) of the respective liposome preparations obtained by the method of the present invention and in Comparative Examples.

Best Modes for Carrying Out the Invention

The present invention will hereinafter be described in further detail.

A liposome preparation is a drug loaded in liposomes. Although there are several methods for the production of liposome preparations, the present invention is a production method of a liposome preparation by a so-called remote loading method that prepares liposomes, into which the incorporation of a drug is intended, beforehand and the drug is then incorporated into the liposomes. Specifically, the present invention is **characterized in that** a specific drug incorporation step (2) to be described subsequently herein is conducted.

No particular limitation is imposed on the liposomes to be provided to the drug incorporation step (2) insofar remote loading for the drug can be conducted, but a description will be made first about the liposomes, into which the incorporation of the drug is intended, and their preparation steps (1).

<Liposomes>

Liposomes are sealed vesicles formed by phospholipid bilayers, and exist in the form of a suspension in which internal water phases in sealed compartments and an external water phase exist isolated from each other by the bilayers. The term "liposomes" as used herein may, therefore, be used with a meaning that encompasses this liposome suspension. As liposome membrane structures, there are known unilamellar vesicles each formed of a lipid bilayer as a single layer and multilamellar vesicles (MLV). These unilamellar vesicles are known to include SUV (Small Unilamellar Vesicle), LUV (Large Unilamellar Vesicle), and the like. No particular limitation is imposed on the membrane structure in the present invention.

A phospholipid is generally an amphiphilic substance having, in its molecule, hydrophobic groups formed of long-chain alkyl groups, and a hydrophilic group formed of a phosphate group. As phospholipids, there can be mentioned glycerophosphoric acids such as phosphatidyl choline (= lecithin), phosphatidyl glycerol, phosphatidic acid, phosphatidyl ethanolamine, phosphatidyl serine, and phosphatidyl inositol; sphingophospholipids such as sphingomyelin (SM); natural or synthetic diphosphatidyl phospholipids, such as cardiolipin, and their derivatives; and their hydrogenated products, for example, hydrogenated soybean phosphatidyl choline (HSPC); and the like. These phospholipids can be used either singly or in combination.

In the present invention, one or more membrane components other than phospholipids may also be contained insofar as they permit stable formation of liposomes. From the standpoint of the phospholipid as a main membrane material, use of a main membrane material having a phase transition point higher than the in vivo temperature (35 to 37°C) is desired to avoid readily leakage of the loaded drug during storage or in the body such as blood. Preferably, the phase transition point of the main membrane material may be 40°C or higher. As phospholipids having such phase transition points, hydrogenated phospholipids such as HSPC, SM and the like are preferred.

The above-described other membrane components may include, for example, lipids free of phosphoric acid (other membrane lipids), membrane stabilizers, antioxidants and the like as needed. As other lipids, fatty acids and the like can be mentioned. As the membrane stabilizers, there can be mentioned, for example, sterols such as cholesterols and saccharides such as glycerol and sucrose, which lower the membrane fluidity. Illustrative of the antioxidants are ascorbic acid, uric acid, tocopherol homologs such as vitamin E, and the like. Tocopherol includes four isomers consisting of $\alpha$-, $\beta$-, $\gamma$- and $\delta$-tocopherols. They are all usable in the present invention.

It is to be noted that in the present invention, the term "a lipid as a liposome membrane component" is used with a meaning which includes all lipids other than drugs, such as phospholipids as main membrane materials, other membrane lipids, and lipids such as sterol as the above-described membrane stabilizers, and further, lipids included in membrane modifiers to be mentioned subsequently herein.

When the total lipid amount of such membrane components is assumed to be 100 mol%, a phospholipid or phospholipids may amount generally to from 20 to 100 mol%, preferably to from 40 to 100 mol%, and other lipid or lipids may amount generally to from 0 to 80 mol%, preferably to from 0 to 60 mol%.

In the present invention, it is also possible to include one or more other membrane modification components, which can retain the membrane structures of liposomes and can be included in the liposome preparation, to extent not impairing the object of the present invention.

As the membrane modification components, hydrophilic polymers and other surface modifiers can be mentioned, for example. No particular limitation is imposed on the time of use of such a membrane modification component insofar as it is used during the liposome preparation step. Concerning the membrane modification with a hydrophilic polymer out of such membrane modification components, the hydrophilic polymer may preferably be selectively distributed in the outer surface of each liposome membrane, especially from the outer membrane of its lipid bilayer to the side of the external medium from the standpoints of the efficiency of distribution and the protection of the hydrophilic polymer from the influence of the drug existing in the internal water phase. In the present invention, it is hence desired to add the hydrophilic polymer after the formation of liposomes, especially after a sizing step.

When a hydrophilic polymer is used as an its lipid derivative, its lipid region(s) as hydrophobic region(s) is (are) held within each membrane so that hydrophilic high-molecular chains can be stably distributed on the outer surface.

Although no particular limitation is imposed on the hydrophilic polymer, illustrative of hydrophilic polymers are polyethylene glycol, polyglycerin, polypropylene glycol, ficoll, polyvinyl alcohol, styrene-maleic anhydride alternating copolymer, divinyl ether-maleic anhydride alternating copolymer, polyvinylpyrrolidone, polyvinyl methyl ether, polyvinylmethyloxazoline, polyethyloxazoline, polyhydroxypropyloxazoline, polyhydroxypropylmethacrylamide, polymethacrylamide, polydimethylacrylamide, polyhydroxypropyl methacrylate, polyhydroxyethyl acrylate, hydroxymethylcellulose, hydroxyethylcellulose, polyaspartamides, synthetic polyamino acids, and the like. In addition, water-soluble polysaccharides such as glucuronic acid, sialic acid, dextran, pullulan, amylose, amylopectin, chitosan, mannan, cyclodextrin, pectin and carrageenan, and their derivatives, for example, glycolipids can also be mentioned.

Among these, polyethylene glycol (PEG) has an effect of improving in-blood retention and is thus preferred. No particular limitation is imposed on the molecular weight of PEG, but its molecular weight may be generally from 500 to 10,000 daltons, preferably from 1,000 to 7,000 daltons, more preferably from 2,000 to 5,000 daltons.

It is to be noted that in the present invention, the term "in-blood retention" means a property that a drug in a state that it is loaded in liposomes of a liposome preparation remains in the blood of a host to which the liposome preparation is administered.

Examples of the lipids (hydrophobic regions) of the hydrophilic high-molecular lipid derivatives include phospholipids, long-chain aliphatic alcohols, sterols, polyoxypropylene alkyl, glyceryl fatty acid esters, and the like. Specifically, a phospholipid derivative or cholesterol derivative of PEG can be mentioned when the hydrophilic polymer is PEG. As the phospholipid, phosphatidyl ethanolamine can be mentioned as a preferred one, and its acyl chain can be generally a saturated fatty acid of $C_{14}$-$C_{20}$ or so, for example, dipalmitoyl, distearoyl, palmitoylstearoyl, or the like. For instance, the distearoylphosphatidyl ethanolamine derivative of PEG (PEG-DSPE) and the like are readily-available, general-purpose compounds.

The percent modification of liposomes by a hydrophilic polymer may be generally from 0.1 to 10 mol%, preferably from 0.1 to 5 mol%, more preferably from 0.2 to 3 mol% in terms of the percentage of the amount of the hydrophilic polymer based on the membranes (total lipid). It is to be noted that the lipid in the lipid derivative of the hydrophilic polymer is also included in this total lipid.

In the present invention, the percent surface modification by such an outer-surface-selective, hydrophilic polymer as described above may be, at a ratio of liposome membrane components to total amount of lipids, generally from 0.1 to 20 mol%, preferably from 0.1 to 5 mol%, more preferably from 0.5 to 5 mol%.

Examples of other surface modifiers include water-soluble polysaccharides such as glucuronic acid, sialic acid, dextran, pullulan, amylose, amylopectin, chitosan, mannan, cyclodextrin, pectin and carrageenan; compounds having acidic functional groups; and basic compounds having basic functional groups such as amino, amidino and guadinino groups. As the basic compounds, there can be mentioned compounds such as DOTMA disclosed in Japanese Patent Laid-Open No. Sho 61-161246, DOTAP disclosed in JP-T-H5-508626, transfectam disclosed in Japanese Patent Laid-Open No. Hei 2-292246, TMAG disclosed in Japanese Patent Laid-Open No. Hei 4-108391, 3,5-dipentadesiloxybenzamidine hydrochloride disclosed in PCT Patent Publication No. WO97/42166, DOSPA, TfxTM-50, DDAB, DC-CHOL, DMRIE, and the like.

When the above-described other surface modifiers are substances formed of lipids and compounds having basic functional groups and bonded thereto, they are called "cationized lipids." The lipid region of such a cationized lipid can be stabilized within the lipid bilayer of each liposome, and its basic functional group region can exist on the surface of the lipid bilayer (on the surface of the outer membrane and/or on the surface of the inner membrane) of the carrier. By modifying the liposome membranes with the cationized lipid, the adhesion or the like between the membranes of liposomes and cells can be enhanced.

(1) Preparation step of liposome suspension

As preparation methods of a liposome suspension, various technologies are known such as the hydration method (the Bangham method), the ultrasonication method, the reverse phase evaporation method (Reverse phase evapolation vesicles), the heating method, the lipid dissolution method, the DRV method (Dehydrated/Rehydrated Vesicles), the freezing-melting method, the ethanol injection method, the thin film method, the extrusion method, and the high-pressure emulsification method by a high-pressure discharge emulsifier (TERADA, YOSHIMURA, et al.: "Liposomes in Life Science" (in Japanese), Springer-Verlag Tokyo (1992)).

As technologies developed in recent years, there are also the jet stream emulsification method that makes use of a velocity change from compression under extreme pressure to perform shear emulsification by a jet stream in a liquid phase, the liposome preparation technology making use of supercritical carbon dioxide, the improved ethanol injection method that can simplify the subsequent sizing step, and so on.

In the present invention, a liposome preparation step (1) typically includes (i) a step of forming liposomes to afford a coarse liposome suspension, (ii) a sizing step for the coarse liposome suspension, and (iv) a step of exchanging the external medium to obtain a liposome suspension, and preferably further includes (iii) a surface modification step with a hydrophilic polymer between the steps (ii) and (iv).

Upon conducting these steps, desired one of preparation methods such as those described above can be adopted as needed. Further, two or more methods can be chosen instead of choosing only one method, and the same method can be repeated or another method can be additionally conducted.

Among the foregoing, the lipid dissolution method is useful as a method oriented toward practical application, and the DRV method is useful as a method for having a drug retained a lot in internal water phases. The method that dissolves a lipid under heat by using, for example, ethanol is widely employed. As liposomes generally have a phase transition point, the respective steps for the preparation of the liposomes before the external medium exchange step (iii) may preferably be conducted at a temperature equal to or higher than the phase transition point of the main membrane material.

It is to be noted that in each of the steps to be described hereinafter, the term "heating temperature" generally means the phase transition point of a lipid, especially a main membrane material or higher. When HSPC is used as a phospholipid of a main membrane material, for example, the heating temperature may be from 50 to 80°C, preferably from 60 to 70°C because its phase transition point is around 50°C. When a formulation containing cholesterol in addition to HSPC is used, however, the phase transition point drops and becomes unclear. In the case of a formulation which contains HSPC and cholesterol in 50 mol% proportions respectively, for example, the phase transition point is around 40°C so that the heating temperature may be from 40 to 70°C, more preferably from 40 to 50°C. As it is necessary to take into consideration the physiochemical stability of the lipid membranes at this time, the heating temperature may desirably be somewhat higher than the phase transition point.

In the preparation step (1) of the liposome suspension, a lipid homogenization step in the liposome formation step (i), the sizing step (ii), and the surface modification step (iii), and further a subsequent drug incorporation step (2) are conducted by making use of the deformation of liposomes and the liquid-crystallization of lipid membranes, and therefore, require the above-described heating temperature. To avoid variations in particle size due to the deformation of the liposomes in the steps other than these steps, however, the external medium exchange step (iv), an unloaded drug elimination step in the drug incorporation step (2) and a final sterilization step may desirably be conducted at a low temperature not higher than the phase transition point of the main membrane material.

The term "low temperature" will hereinafter means preferably from 0 to 40°C or so, typically from 5 to 25°C or so when the phase transition point of the main membrane material is, for example, around 50°C.

The present invention will hereinafter be described based on preferred embodiments, although the present invention shall not be limited specifically to the following embodiments.

In the liposome formation step (i), membrane components such as those described above and a water phase are mixed to form liposomes so that a coarse liposome suspension is afforded. As the water phase to be mixed with the membrane components, an aqueous solution with an osmoregulator, pH adjuster and the like dissolved as needed in injection-grade water may be used generally.

When forming liposomes by using another lipid as a membrane stabilizer along with the phospholipid in the step (i), a homogenization step may desirably be conducted before the mixing of the membrane components and the water phase to avoid heterogenization which would otherwise be caused by the plural lipids. No particular limitation is imposed on this homogenization step. In general, however, the membrane components, especially the lipids are completely dissolved in an organic solvent to prepare a lipid solution. As the organic solvent, one having volatility such as, for example, chloroform or ethanol, is generally employed. From the standpoint of a practical application on industrial scale, ethanol, particularly absolute ethanol is preferred. When this dissolution is conducted at a heating temperature equal to or higher than the phase transition point of the main membrane material, a homogeneous lipid solution can be obtained more easily.

For the mixing of the membrane component and the water phase, the aqueous solution and the homogenized lipid solution may be stirred, for example, after heating them to the above-described heating temperature beforehand. For this stirring, a stirrer such as a propeller stirrer may be used in general. This stirring may also be conducted at the above-described heating temperature as needed.

Particle size control of the coarse liposome suspension, namely, the sizing step (ii) is next conducted to obtain a sized liposome solution. To perform sizing of liposomes to a desired size, various known technologies may be used including membrane emulsification and maintenance of shear force. There are, for example, the above-exemplified high-pressure emulsification method, the membrane emulsification method that forcedly pass the coarse liposome suspension a plurality of times through a filter, etc. These methods are all described in G. Gregoriadis: "Liposome Technology Liposome Preparation and Related Techniques," 2nd edition, Vol. I-III, CRC Press.

When the sizing step is conducted, for example, by the membrane emulsification method, the particle size can be controlled using a membrane filer such as a commercially-available polycarbonate membrane filter. When desired to control the particle size to 100 nm, sizing can generally be performed stepwise by combining membrane filters such as 400 nm, 200 nm and 100 nm membrane filters.

The particle size control is easy when the coarse liposome suspension is equal to or higher than the phase transition point of the main membrane material in the sizing step.

No particular limitation is imposed on the size of the liposomes after the sizing. When the liposomes take a spherical shape or a shape close to the spherical shape, however, the diameters of the particle profiles are generally from 0.02 to 2 μm, preferably from 0.05 to 0.25 μm. Using a Zetasizer (Malvern Instruments, 3000HS), this particle size is measured as the average diameter value of the entire particles by the dynamic light scattering method.

The liposomes sized as described above are preferably subjected to surface modification before the external medium exchange step (iv). As mentioned above, the surface modification step (iii) is conducted with a view to imparting a function to the outer surfaces of the liposomes, and is carried out by modifying the outer surfaces of the liposomes, for example, with a ligand such as an antibody for imparting a targeting function or with a hydrophilic polymer having a function to improve the in-blood retention. Liposomes modified at the outer surfaces thereof with a surface modifier such as a hydrophilic polymer can be provided with physiochemical stability in the production process so that they can be stable treated even under conditions such as heating and cooling during their production. From the standpoint of obtaining physiochemical stability such as liposome particle size in subsequent steps such as the external medium exchange step (iv) and further, the drug incorporation step (2), it is hence desired to conduct the surface modification step (iii) before these steps, specifically immediately after the liposome sizing step (ii).

Specifically, the surface modification mixes the sized liposome solution and a surface modifier solution, and then stirs them at the heating temperature. By referring the surface modifier solution as a "hydrophilic polymer solution" for the sake of convenience, a description will hereinafter be made about an example that typically uses a lipid derivative of a hydrophilic polymer as a surface modifier, although the following description similarly applies to cases where other surface modifications are performed. The mixing of the sized liposome solution and the hydrophilic polymer solution is feasible from immediately after the completion of the sizing step, and the time until the mixing may preferably be as short as permissible. Preferably, the time until the mixing may be within 180 minutes at the latest from immediately after the completion of the preceding step.

The hydrophilic polymer may be used as an aqueous solution as needed. In general, the hydrophilic polymer and sized liposomes may each be maintained preferably in a state heated equal to or higher than the temperature at which the sizing was conducted, that is, the phase transition point of the main membrane material.

As a mixing method, the hydrophilic polymer solution may be added to the sized liposome solution, or vice versa. It is preferred to conduct the mixing under stirring. For the stirring, a stirrer such as a propeller stirrer can be used generally.

The surface modification step (iii) can be conducted using a heat exchanger. As the liposomes after the sizing are unstable, it is desired to conduct the heating in a time not impairing physiochemical stability such as the particle size. From the standpoint of shortening the time, it is also desired to conduct the surface modification step by using a heat exchanger. Details of a method for conducting the surface treatment by using a heat exchanger are described, for example, in Japanese Patent No. 2766691. Because the liposomes immediately after the sizing are unstable, the sized liposome solution can be cooled once through a heat exchanger to or lower than the transition point, and can then be heated again through another heat exchanger to conduct the surface modification step under heat.

As described above, the surface modification step is generally conducted at a heating temperature equal to or higher than the phase transition point of the main membrane material. The present inventors have, however, been found that, when an alcohol exists in the surface modification step, the incorporation time of the hydrophilic polymer can be significantly shortened, and moreover, the condition for the incorporation temperature can be lowered. For the liposomes after the surface modification step, it is desired, from the standpoint of the stability of lipids, to lower the temperature condition as much as possible and to shorten the heating time as much as possible. Significant advantages can, therefore, be brought about from the feasibility of conducting the surface modification step at a low temperature. For making an alcohol exist in the surface modification step, it is most desired to use the alcohol for the homogenization of lipids in the liposome formation step as the use of the alcohol also makes it possible to most conveniently conduct the homogenization of the lipids.

After the surface modification step (iii), physiochemical stability such as the particle size can be assured, but no assurance can be made for chemical stability such as hydrolysis of lipids. From the standpoint of ensuring chemical stability, it is desired to reduce the thermal exposure time in all the steps for the production of liposomes and a liposome preparation. From the standpoint of this stability of lipids, a shorter heating time is desired, and after the surface modification, the liposomes may desirably be cooled promptly. As a simpler and more convenient cooling method, ice-cooling is preferred. As a method for promptly cooling cooling the liposome after the completion of the surface modification step, the above-described method that uses a heat exchanger is also suited.

It is to be noted that the unbonded surface modifier can be eliminated in a subsequent unloaded-drug elimination step. From this standpoint, it is desired to include the unloaded drug elimination step after the surface modification step.

In the external medium exchange step (iv), exchange of the external medium is conducted for remote loading.

The sized and surface-modified liposomes make it possible to stably conduct the subsequent steps so that they can be conducted under conditions known in common. The external medium exchange step (iv) may preferably be conducted at the above-described low temperature to avoid an application of unnecessary heat. Described specifically, when the phase transition point of the main membrane material is around 50°C, for example, 0 to 40°C or so, typically 5 to 25°C or so is preferred. In this step, it is possible to conduct elimination of the alcohol brought in from the liposome formation step (i) and the hydrophilic polymer not incorporated into the liposomes in the surface modification step (iii). It is to be noted that upon preparing a liposome preparation at need in a hospital ward, the production of the liposome preparation provides a final preparation through a sterilization step after the above step (iii) and subjects this final preparation to the drug incorporation step (2) at the time of the preparation at need in the hospital ward.

The external medium exchange step (iv) exchanges the external water phase out of the internal and external water phases of the liposomes formed of the water phases, which was employed in the liposome formation step (i), to obtain a liposome suspension. As methods for exchanging the external water phase, there are the dialysis method, the ultracentrifugal separation method, the gel filtration method, and the like. These methods are all described in G. Gregoriadis: "Liposome Technology Liposome Preparation and Related Techniques," 2nd edition, Vol. I-III, CRC Press. Especially as a production method making use of medium- or large-scale facilities to achieve practical application, there can be mentioned a method making use of hollow fibers such as a dialyzer, tangential flow making use of ultrafiltration membranes, diafiltration, and the like.

Primary purposes of the external medium exchange step (iv) in the present invention are to eliminate an organic solvent brought in at the formation step (i) and to form an ion gradient across the internal and external phases of the liposomes to permit remote loading at the drug incorporation step (2). When the surface modification step (iii) is conducted as a preceding step, the external medium exchange step (iv) is also useful as a step for the elimination of the surface modifier not bonded to the liposomes.

The remote loading method can be used for common drugs each of which can exist in a charged state when dissolved in an appropriate aqueous medium. Typically, the formation of an ion gradient across the interiors and exteriors of the liposomes allows a drug to permeate through the liposome membranes in accordance with the thus-formed gradient so that the drug can be loaded into the liposomes. No particular limitation is imposed on the type of the gradient in the present invention insofar as a desired drug can be incorporated into liposomes prepared beforehand. Some specific examples of remote loading will be described hereinafter.

As an ion gradient to be formed across liposome membranes, there is a $Na^+/K^+$ concentration gradient. A technology that adds a drug into preformed liposomes by a remote loading method for a $Na^+/K^+$ concentration gradient is disclosed in JP-T-H07-112968, etc., and the incorporation of a drug can be conducted with reference to this document.

In the present invention, a proton concentration gradient can be preferably mentioned as the ion gradient. There can be mentioned an embodiment having a pH gradient that the internal (internal water phase) pH of liposome membranes is lower than the external (external water phase) pH. A pH ingredient can be formed, specifically, by an ammonium ion ingredient and/or a concentration ingredient of an organic compound having one or more protonizable amino groups.

As a specific example of the method that loads a drug into liposomes by an ammonium ion concentration gradient, liposomes are firstly formed beforehand in an aqueous buffer containing from 0.1 to 0.3 M of an ammonium salt, and the external medium is exchanged with a medium containing no ammonium ions, for example, a sucrose solution to form an ammonium ion gradient between the interiors and exterior of the liposome membranes. Internal ammonium ions are equilibrated by ammonia and protons, and the ammonia diffuses through the lipid films and are eliminated from the interiors of the liposomes. Along with the elimination of the ammonia, the equilibrium inside the liposomes continuously shifts toward the formation of protons. As a result, protons are accumulated within the liposomes, and therefore, a pH gradient is formed between the interiors and exterior of the liposomes. By adding the drug to the liposome suspension having this pH gradient, the drug is loaded into the liposomes.

No particular limitation is imposed on the ammonium salt that can form an ammonium ion concentration gradient, but there can be mentioned ammonium sulfate, ammonium hydroxide, ammonium acetate, ammonium chloride, ammonium phosphate, ammonium citrate, ammonium succinate, ammonium lactobionate, ammonium carbonate, ammonium tartrate, ammonium oxalate, and the like.

A technology that incorporates a drug into preformed liposomes by a remote loading method for an ammonium ion concentration gradient is disclosed in U.S. Patent No. 5192549, and the incorporation of a drug can be conducted with reference to this document.

As the organic compound having one or more protonizable amino groups, one having a lower molecular weight is desired. Specifically, methylamine, ethylamine, propylamine, diethylamine, ethylenediamine, aminoethanol or the like can be mentioned, although the organic compound is not limited to it.

(2) Drug incorporation step

Using the liposome suspension after the external medium exchange step (iv), the drug is incorporated by the remote loading method in the present invention. The drug is generally incorporated by a drug solution.

It is to be noted that the expression "to load a drug into liposomes" means to have the drug contained as internal water phases or in internal water phases and that the term "liposome preparation" means liposomes with the drug loaded therein. The term "loaded" means that the drug is held by adhesion on the membranes or is held within the membranes, and in this case, is not necessarily limited to the existence of the drug in a state that it is dissolved in the internal water phases.

The drug incorporation step (2) in the present invention is conducted at or higher than the phase transition point of the liposome lipid membranes. The present invention is **characterized in that** this drug incorporation step (2) is conducted using rapid heating means. Described specifically, the mixture of the liposome suspension and the drug solution is fully heated in a short time to or higher than the phase transition point of the lipid membranes. The rapid heating means has been chosen from the standpoint of production time, from the standpoint of quality such as uniformity and from the standpoint of the physiochemical stability of the preparation.

Employed in conventional methods is the method that stirs and mixes two solutions of a liposome suspension and a drug solution, both of which have been heated to or higher than the phase transition point, in a vessel. In a medium- or large-scale production method, however, the above-mentioned method is not preferred from the standpoint of the physiochemical stability of a drug and liposomes because enormous time is needed for simply preheating and mixing these two solutions. When mixing the preheated two solutions with each other, the greater the production scale, the longer the time required for the mixing. It has also been found that liposomes before the incorporation of a drug, the liposomes having a pH gradient formed thereon, are more susceptible to lipidolysis compared with the liposomes after the incorporation of the drug. Considering in view of this finding, it is not preferred to include a step that provisionally heats liposomes before the incorporation of a drug. Furthermore, in the course of arriving at the present invention, the present inventors also found that the incorporation of a drug takes place in one minute when the two solutions are heated to or higher than the phase transition point of the lipid membranes. In view of this finding, it has been found that, when the two solutions heated to or higher than the phase transition point of the lipid membranes are mixed together using the conventional method, the incorporation of the drug begins from the time of the commencement of the mixing and a uniform percent incorporation of the drug can be hardly obtained among preparations.

Described specifically, the liposome suspension is transferred into a storage tank in the drug incorporation step in the present invention while maintaining it at or lower than the phase transition point of the lipid(s) which constitute(s) the liposome membranes. Before feeding the liposome suspension and drug suspension to the rapid heating means, the liposome suspension may preferably be mixed and stirred with the drug solution still at a temperature equal to or lower than the phase transition point of the lipid membranes to homogenize the drug solution and the liposome suspension beforehand. As this mixing is conducted at or below the phase transition point of the lipid membranes, no incorporation of the drug takes place. Therefore, in the mixture, the drug can be evenly distributed in the external medium of the liposomes. The temperature of the mixture may be preferably from 5 to 35°C, more preferably from 10 to 30°C, because an unduly low temperature results in difficult temperature control and cumbersome work while an excessively high temperature may induce the incorporation of the drug into the liposomes even at a temperature lower than the phase transition point of the lipid membranes.

The rapid heating equipment can rapidly heat the mixture to a predetermined temperature. The heating rate may be from 1 to 20°C/sec, preferably from 3 to 10°C/sec. As the rapid heating equipment, a heat exchanger is used. The heat exchanger can rapidly heat the mixture to the predetermined temperature, and can also readily maintain the heated temperature for a predetermined time.

Specifically, the above-described heating method instantaneously heats a mixture in a short time in the course of its feeding to load the drug.

The heat exchanger for use in the heating step is to perform an exchange of heat between two fluids through a partition, and is used for applications such as heating, evaporation, cooling and condensation.

In the present invention, the use of the heat exchanger as the heating means in the drug incorporation step makes effective use of this characteristic. In general, there are well known multitubular cylindrical (capillary) heat exchangers, double-pipe heat exchangers, plate heat exchangers, coil heat exchangers, spiral heat exchangers, jacketed heat exchangers, nonmetal heat exchangers, etc. There are also heating furnaces in each of which one of fluids is fed to an open system, thermal storage heat exchangers (in each of which low-temperature and high-temperature fluids are alternately brought into contact with a solid to effect a heat exchange), and the like.

No particular limitation is imposed on the heat exchanger, but a multitubular cylindrical heat exchanger, a double-pipe heat exchanger, a plate heat exchanger or the like is preferred. The greater the heat transfer area ($m^2$) in a heat exchanger, the higher the heat efficiency. In the case of, for example, a multitubular cylindrical heat exchanger, however, consideration is needed from the standpoint of cleaning or the like as the piping becomes too narrow.

As a preferred embodiment, a heat exchanger may be arranged for the purpose of heating before the drug incorporation step which should be conducted under heat, and another heat exchanger may also be arranged for the purpose of cooling as a subsequent cooling step. As this heat exchanger for the purpose of cooling, it is possible to use an air cooler (cooling by air) or an irrigation cooler (cooling by water spray) as an alternative for the above-exemplified heat exchanger. The use of a rapid heating equipment such as a heat exchanger in the drug incorporation step can achieve shortening of time, uniformity improvements in production and improvements in physiochemical stability uniformity, and therefore, is effective.

In the drug incorporation step according to the present invention, such heat exchanger is used to rapidly raise the temperature of the drug-liposome mixture, which has been held not higher than the phase transition point of the membrane lipids, to a preset temperature equal to or higher than the phase transition point, so that the liposome membranes is provided with drug permeability to incorporate the drug into the liposomes by the above-mentioned remote loading method. As rapid heating means such as a heat exchanger is used for the heating, heating in an extremely short time is feasible, and this heating can be effected fully and evenly. Because the drug exists in the same amount around the respective liposome particles at the time of the heating, it is possible to obtain a liposome preparation having a uniform amount of incorporated drug.

In the present invention, the temperature of heating by the rapid heating means is supposed to be equal to or higher than the phase transition point of the membrane lipid(s) that form(s) the liposomes, and may be set higher preferably by from 0 to 40°C, more preferably by from 5 to 30°C, still more preferably by from 10 to 20°C than the phase transition point. When it is higher by 5°C or more, the liposomes are provided with sufficient drug permeability so that the incorporation of the drug by the rapid heating means can be effectively conducted. When it is not higher than 40°C, the membrane lipid(s) or drug is provided with stability. More specific temperature values are set to avoid readily leakage at body temperature when administration into the blood is intended, although they vary depending on the phase transition point of membrane lipid(s) to be used. As the phase transition point is from 35 to 40°C or so, the heating temperature may be set at preferably from 40 to 80°C, more preferably from 45 to 70°C, still more preferably from 50 to 60°C. It is to be noted that, at a temperature lower than 40°C, the liposome membranes can hardly be provided with sufficient drug permeability. At a temperature higher than 80°C, on the other hand, there is a potential problem in that an adverse effect may be given to the membrane lipid(s) or drug even when the temperature lasts in a short time.

Further, the heating time by the heat exchanger, in other words, the residence time in the heat exchanger may be preferably from 3 to 120 seconds, more preferably from 5 to 30 seconds. The term "residence time in a heat exchanger" means the time of passage from the inlet to the outlet of the heat exchanger. This residence time in a heat exchanger varies depending on the effective internal cross-sectional area of its tube and the flow rate.

When the rapid heating means is a heat exchanger as in the present invention, the drug can be continuously incorporated unlike the conventional methods. Described specifically, interposition of a heat exchanger in a feed pipe makes it possible to incorporate the drug during the feeding from the storage tank employed in the drug solution homogenization step to another storage tank, and therefore, can substantially shorten the time compared with the conventional methods.

In the remote loading method, the pH of the internal water phase is generally lower so that hydrolysis of the lipid(s) tends to occur under exposure to heat. From this standpoint, use of a temperature down step is desired to avoid thermal exposure as much as possible. The preset temperature in the temperature down step may be not higher than the phase transition point of the lipid membranes, preferably from 1 to 25°C, more preferably from 5 to 10°C. An unduly low temperature results in difficult temperature control and cumbersome work while an excessively high temperature may induce the incorporation of the drug into the liposomes even at a temperature lower than the phase transition point of the lipid membranes. As one example of the temperature down step, the temperature of a liposome suspension discharged from a heat exchanger can be lowered by submerging a storage tank, in which the liposome suspension is to be stored, in ice water and ice-cooling the storage tank. To reduce the thermal exposure time of the drug and lipid membranes as much as possible, shortening of the period of residual heat after the heating is also an important issue. With a view to more positively shortening the period of residual heat, it is possible to use not only a heat exchanger for a temperature rise but also a heat exchanger for a temperature down. The liposome suspension after the incorporation of the drug can be promptly cooled, for example, by interposing an additional heat exchanger for a temperature down in a feed pipe on a downstream side of a heat exchanger for drug incorporation.

The drug incorporation step (2) has been described above based on the example that it is conducted after the external medium exchange step (iv). However, the drug incorporation step (2) may be conducted at any other suitable timing in some instances. For example, it may be applied upon preparation at need in a hospital ward. A preparation to be used for the preparation at need in the hospital ward may desirably be one gone through the external medium exchange step in the remote loading method and having an ion gradient formed therein.

Described specifically, the incorporation of a drug by making use of a heat exchanger can be used in preparation at need in a hospital ward when the thermal stability of the drug and its stability in an aqueous solution are extremely low. There can be mentioned, for example, a method that dissolves the drug at a temperature lower than the phase transition point in a liposome suspension after the external medium exchange and internally arranges a heat exchanger in a step in the course of feeding of the liposome suspension to a drip infusion route to heat the liposome suspension to or above the phase transition point. The heat exchanger in this case may desirably be, for example, of the small and disposal type from the standpoint of the use in the hospital ward. As a dissolving and mixing method in this case, the drug may be mixed after dissolving it in another dissolving agent or may be directly dissolved with an empty liposome suspension. Further, after conducting the incorporation of the drug under heat as mentioned above, the drug-incorporated liposome suspension may be cooled to promptly lower its temperature.

The present invention can be applied to various drugs. As drugs for therapy, for example, there can be mentioned nucleic acids, polynuleotides, genes and analogs thereof, anticancer agents, antibiotics, enzymes, antioxidants, lipid intake inhibitors, hormones, antiinflammatories, steroids, vasodilators, angiotensin converting enzyme inhibitors, angiotensin receptor antagonists, smooth myocyte growth/migration inhibitors, platelet aggregation inhibitors, anticoagulants, chemical mediator t release inhibitors, endothelial cell growth promoters or inhibitors, aldose reductase inhibitors, mesangium cell growth inhibitors, lipoxygenase inhibitors, immunosuppressives, immunostimulants, antiviral agents, Maillard reaction suppressors, amyloidosis inhibitors, nitrogen monoxide synthesis inhibitors, AGFs (Advanced glycation endproducts) inhibitors, radical scavengers, proteins, peptides, glycosaminoglycans and derivatives thereof, oligosaccharides, polysaccharides, and the like. Specifically, there can be mentioned adrenal cortical steroids such as prednisolone, methylprednisolone and dexamethasone, and their derivatives, nonsteroidal antiinflammatory drugs such as aspirin, indometacin, ibuprofen, mefenamic acid and phenylbutazone, mesangium cell growth inhibitors such as heparin and low molecular heparin, immunosuppressives such as cyclosporin, ACE (angiotensin converting enzyme) inhibitors such as captopril, AGE (advanced glycation endoproduct) inhibitors such as methylguanidine, β antagonists such as biglycan and decorin, PKC (protein kinase C) inhibitors, prostaglandin preparations such as PGE and PGI, peripheral vasodilators such as papaverine drugs, nicotinic acid drugs, tocopherol drugs and Ca antagonists, antithrombotics such as phosphodiesterase inhibitors, ticlopidine and aspirin, anticoagulants such as warfarin, heparin and antithrombin agents, thrombolytics such as urokinase, chemical mediator release inhibitors, antibiotics, antioxidants, enzymes, lipid intake inhibitors, hormones, radical scavengers such as vitamin C, vitamin E and SOD, antisense oligonucleotides having mesangium cell growth inhibiting activity, decoys, genes, and the like.

As drugs for diagnosis, on the other hand, there can be mentioned in vivo diagnostic media such as X-ray contrast media, ultrasonic diagnostic media, diagnostic media for radioisotope-labeled nuclear medicine and diagnostic media for nuclear magnetic resonance diagnosis. The use of the process according to the present invention is suited especially upon loading drugs which are unstable in aqueous solutions, nucleic acids, polynucleotides, genes and the like.

For a drug such as that described above, a high percent load is generally desired, although the desired percent load differs depending on the kind of the,drug. According to the incorporation of a drug by the remote loading method, a high drug/lipid(s) can be achieved, thereby making it possible to obtain a liposome preparation having a high percent load and effective for clinical applications.

In the liposome preparation according to the present invention, a high percent drug load of preferably 80% or higher, preferably 90% or higher can be achieved. This percent drug load is a value available as an average value. Nonetheless, such a high percent load means small variations in percent load among individual liposome preparations, and hence, a uniform percent load in all the preparations.

The liposome suspension after the above-described drug incorporation step is subjected to steps known in common such as the unloaded drug elimination step and the sterilization step.

The unloaded drug elimination step has for its object of the elimination of the unloaded drug after the drug incorporation step. Basically, an operation similar to the external medium exchange step is conducted, but the unloaded drug elimination step is different in object, that is, in that it eliminates the drug remaining in the external water phase.

The sterilization step conducts sterilization after the liposome formation step. No particular limitation is imposed on the method of sterilization, and usable examples include filter sterilization, autoclave sterilization, dry-heat sterilization, ethylene oxide gas sterilization, radiation (e.g., electron beam, x-ray, γ-ray, or the like) sterilization, sterilization with ozone water, and sterilization making use of hydrogen peroxide solution.

In the present invention, filter sterilization is most preferred as the sterilization step. In filter sterilization, it is required to pass the liposomes but to prevent filtration of Brevundimonas diminuta (size: approx. $0.3 \times 0.8$ μm) to be used as an indicator microorganism. The liposomes are, therefore, required to be substantially smaller particles compared with Brevundimonas diminuta. A particle size of around 100 nm is also important for further ensuring the filter sterilization step.

It is to be noted that this sterilization step may be omitted depending on the production method.

The final preparation obtained through the above-described step can be stored at room temperature (generally from 21°C to 25°C), preferably under refrigeration at from 0 to 8°C from the standpoints of the stability of lipid(s) and the physiochemical stability such as particle size.

As the thermal history of the liposome preparation according to the present invention in the production process is minimized as much as possible, the degradation of the lipid(s) in the liposome preparation can be inhibited, and therefore, the liposome preparation can be provided with excellent storage stability. This storage stability can be assessed in terms of the percent formation of lyso derivative(s) of lipid(s).

## Examples

Examples will next be given to describe the present invention in further detail, but the present invention shall not be limited to these Examples.

### (Preparation Examples 1 to 8) Preparation of liposome preparations

#### (1) Preparation of liposome suspensions

<Formation of liposomes>

Hydrogenated soybean phosphatidyl choline (HSPC, molecular weight: 790, product of Lipoid GmbH, "SPC3") (70.53 g) and cholesterol (Chol, molecular weight: 386.65, product of Solvay S.A.) (29.50 g) were weighed, followed by the addition of absolute ethanol (100 mL) to dissolve them under heat.

To an aliquot (100 mL) of the resultant lipid solution in ethanol, a 250 mM solution of ammonium sulfate (900 mL) which had been heated to approx. 70°C was added, and the resulting mixture was stirred to prepare a coarse liposome suspension. Using an extruder (manufactured by Lipex Biomembranes Inc.) heated at about 65°C, the coarse liposome suspension was passed five times through a filter of 100 nm pore size (polycarbonate membranes) to obtain a liposome suspension.

<Surface modification>

While maintaining the thus-obtained liposome suspension in the heated state, a solution of polyethylene glycol 5000-phosphatidiyl ethanolamine ($PEG_{5000}$-DSPE, molecular weight: 5938, product of NOF Corporation) (7.69 g) in injection-grade water (200 mL) was immediately added to give a PEG incorporation rate of 0.75 mol%, followed by stirring under heat to modify the membrane surfaces (outer surfaces) of the liposomes with PEG. The liposome suspension after completion of the heating was promptly ice-cooled.

It is to be noted that the PEG incorporation rate (mol%) is expressed by the following formula: PEG Incorporation Rate (mol%) = ($PEG_{5000}$-DSPE/Total Lipid) $\times$ 100, in which the total lipid means the total amount of lipid components that form the liposome membranes (HSPC + Chol + $PEG_{5000}$-DSPE). Each lipid component was quantitated by a high performance liquid chromatograph (HPLC).

<External medium exchange>

Using a filter (a membrane crossflow system manufactured by Sartorius AG: "SARTOCON SLICE" (molecular weight cut-off: 300 K)), the dispersion medium (external water phase) of the suspension of the PEG-modified liposomes was subjected to an external medium exchange with a 10% solution of sucrose in 10 mM Tris (pH 9.0). At that time, the external water phase was fed in an amount equal to the amount of the discharged solution such that the concentration of the liposome suspension always remained constant. The liposome suspension after the external medium exchange was ice-cooled.

#### (2) Drug incorporation step

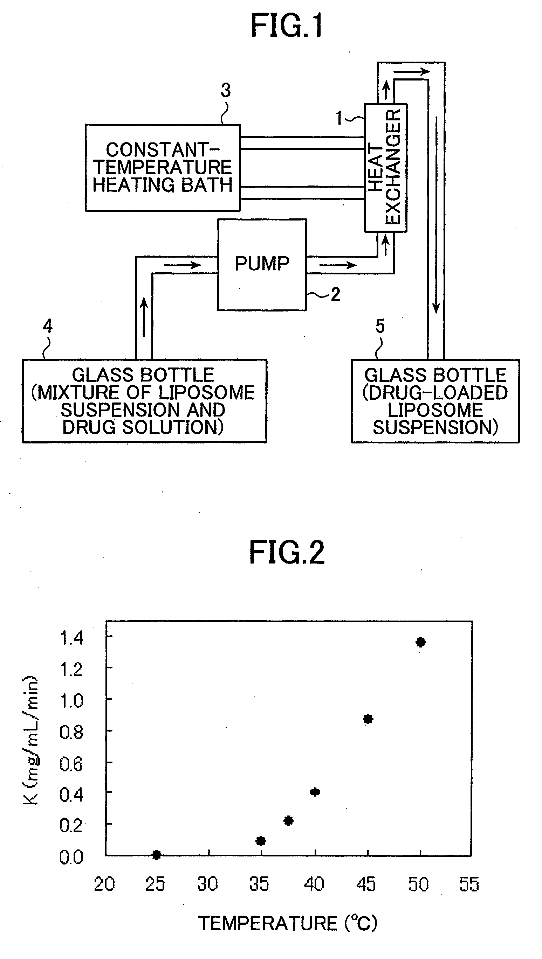

In this preparation example, the incorporation of a drug was conducted using a heat exchanger as rapid heating means. A schematic diagram for describing a drug incorporation step in this embodiment is shown in FIG. 1. Used as a heat exchanger 1 in this embodiment was a multitubular cylindrical heat exchanger made of stainless steel ("CAPIOX (R) CARDIOPREGEAR CX-CP50," manufactured by TERUMO Kabushiki Kaisha; inner tube diameter: 1 mm, effective internal cross-sectional area: 640 $cm^2$).

A mixture of the liposome suspension and a drug solution in a glass bottle 4 was fed into the heat exchanger 1 via a pump 2 such that the mixture flew at a constant rate through the heat exchanger 1. The heat exchanger 1 was heated by a constant-temperature heating bath 3. A drug-incorporated liposome suspension delivered from the heat exchanger 1 was received in a glass bottle 5.

<Preparation of drug solution>

The lipid (HSPC) in the liposomes after the external medium exchange was quantitated by the high performance liquid chromatograph. Based on the concentration of total lipid as calculated from the quantitated HSPC, a calculation was made to determine the amount of Dox (doxorubicin hydrochloride, molecular weight: 579.99) that gave a Dox/total lipid (mol/mol) of 0.16. Based on the calculation results, a necessary amount of Dox was weighed. Using a 10% solution of sucrose in 10 mM Tris (pH 9.0), a 10 mg/ML solution of Dox (drug solution) was prepared.

<Study 1 on drug incorporation by remote loading method>

Using the liposome suspension and drug solution, the drug incorporation rates at respective temperatures (25, 35, 37.5, 40, 45 and 50°C) by the remote loading method were simulatively investigated in small-capacity reaction vessels.

In 50-mL glass vessels, aliquots (8 mL) of the Dox solution (doxorubicin hydrochloride/total lipid = 0.16 mol/mol, 10 mg/mL), the aliquots having been heated beforehand to the respective temperatures, were added to aliquots (15 mL) of the liposome suspension, the aliquots having been heated beforehand to the corresponding temperatures, respectively. Aliquots of each of the resulting mixtures were incubated for 1, 2.5, 5, 7.5, 10 and 20 minutes. The concentration of the unloaded drug at each temperature was determined. Further, presuming that the permeation of a drug follows a first-order kinetics, a function expressed by the following formula was determined, and its gradient was employed as a drug incorporation rate constant.

$$C = C_0 \cdot EXP(-k \cdot t)$$

where, C: Concentration (mg/mL) of unloaded drug (Dox)
$C_0$: Initial concentration (mg/mL) of unloaded drug (Dox)
t: Time (min)
k: Drug incorporation rate constant (mg/mL/min)

The results of the drug incorporation rate constants for the respective temperatures are shown in FIG. 2. It has been found that the drug incorporation rate sharply increases at and above around 35°C. Accordingly, the phase transition point of the liposome membranes employed in this embodiment is gathered to exist around 35°C, and the drug incorporation step needs to be conducted at or above 35°C.

<Study 2 on drug incorporation by remote loading method>

In the above-described study 1, the percent drug incorporation for the respective incubation times at the incubation temperature of 50°C were determined to investigate the time required for the incorporation of the drug.

After physiological saline was added to each drug-loaded liposome suspension to dilute the suspension 20-fold, the thus-diluted suspension was ultracentrifuged ($1 \times 10^5$ g, 2 hours, 10°C) to settle the drug-loaded liposomes, and the corresponding percent drug incorporation (Dox loading efficiency) was determined from the concentration (mg/mL) of the drug in the supernatant in accordance with the below-described formula.

The percent drug incorporation at 50°C is plotted as a function of the incubation time in FIG. 3. It was ascertained that at the incubation temperature of 50°C, the percent drug incorporation was substantially saturated in the shortest time (the arrow in the diagram) among the tested times.

[Calculation formula 1]

$$\text{Percent drug incorporation (\%)} = \frac{\text{Concentration of drug in the whole volume (mg/mL)} - \text{Concentration of drug in supernatant after ultracentrifugation (mg/mL)}}{\text{Concentration of drug in the whole volume (mg/mL)}} \times 100$$

The concentrations (mg/mL) of Dox in the supernatant (unloaded) and the whole volume are quantitative analysis data by fluorometry (Ex 480 nm, Em 580 nm). A calibration line was prepared from aliquots of physiological saline, which contained Dox at the respective concentrations.

<Drug incorporation>

As shown in FIG. 1, the liposome suspension and the drug solution were mixed with each other at approx. 25°C in the glass bottle 4. Aliquots (400 mL) of the resulting mixture were fed into the heat exchanger 1 at the respective flow rates shown in Table 1. Those flow rates were determined in accordance with the following formula.

[Calculation formula 2]

$$\text{Flow rate (g/s)} = \frac{\text{Total weight (g) of the mixture transferred from the glass bottle 4 to the glass bottle 5 through the heat exchanger}}{\text{Time (s) required for the transfer of the mixture from the glass bottle 4 to the glass bottle 5}}$$

The glass bottle 5 which contained the drug-loaded liposome suspension delivered from the heat exchanger 1 was ice-cooled.

Using a column ("Sepharose 4 Fast Flow," 02.8 cm × 20 cm) substituted fully with a 10% solution of sucrose in 10 mM Tris (pH 9.0), elimination of the unloaded drug was then conducted to eliminate the drug which was not loaded in the liposomes.

Finally, a sterilization step was conducted by passing the liposome suspension, which after the unloaded drug elimination, through a 0.2 μm sterilization filter.

Flow rates and residence times of the respective aliquots of the mixture through the heat exchanger 1, preset temperatures of the constant-temperature bath 3, outlet temperatures of the heat exchanger 1, and percent drug incorporations are shown in Table 1. The percent drug incorporation is shown as a function of the outlet of the heat exchanger 1 in FIG. 4. Heating temperatures by the heat exchanger as rapid heating means, that is, temperatures inside the heat exchanger were assumed to be equal to the outlet temperatures of the heat exchanger.

[Table 1]

| | Flow rate (g/s) | Residence time in heat exchanger (s) | Preset temp. of constant-temperature bath (°C) | Outlet temp. of heat exchanger (°C) | Percent drug incorporation (%) |
|---|---|---|---|---|---|
| Preparation Example 1 | 4.0 | 9.9 | 55 | 47.8 | 94.8 |
| Preparation Example 2 | 6.2 | 6.4 | 60 | 49.0 | 97.1 |
| Preparation Example 3 | 8.5 | 4.7 | | 47.0 | 92.4 |
| Preparation Example 4 | 11.5 | 3.4 | 65 | 46.8 | 96.3 |
| Preparation Example 5 | 11.5 | 3.4 | 50 | 38.0 | 22.8 |
| Preparation Example 6 | 11.5 | 3.4 | 55 | 42.0 | 52.1 |
| Preparation Example 7 | 8.5 | 4.7 | | 44.0 | 55.3 |
| Preparation Example 8 | 4.0 | 9.9 | 50 | 44.5 | 63.4 |

As shown in Table 1, the residence times in the heat exchanger, which correspond to the flow ranges of from 4.0 to 11.5 g/s, are from 9.9 to 3.4 seconds. When the outlet temperature of the heat exchanger was higher than 45°C, the loading efficiency of the drug (Dox) exceeded 90%. Different from the conventional method requiring such a long preheating time as shown in Comparative Example 1 to be described subsequently herein, it has been demonstrated that according to the present invention, high loading efficiency can be achieved in an extremely short time without preheating of a drug solution and a liposome suspension before they are brought into contact with each other.

On the other hand, even with the same residence time of 3.4 seconds, an outlet temperature of the heat exchanger lower than 40°C resulted in a low drug loading efficiency at the level of 20% so that no sufficient loading efficiency was available. Further, insofar as the outlet temperature of the heat exchanger was equal to or higher than 40°C, a loading efficiency of the drug (Dox) at the level of from 50 to 60% was successfully obtained by setting relatively long the residence time in the heat exchanger.

From the foregoing, it has been confirmed that, when a mixture obtained by combining the liposome suspension and drug suspension of this Example at a temperature at which incorporation of the drug hardly takes place is heated to a temperature higher than 45°C in a moment by using a heat exchanger, a high loading efficiency of 90% or more can be obtained even in an extremely short time (approx. 3 to 10 seconds), in other words, the incorporation of the drug to a desired concentration can be completed in a short time during feeding through the heat exchanger without preheating.

Because the drug can be incorporated in a short time as described above, it is possible to considerably shorten the time required for the drug incorporation step, and moreover, to substantially reduce thermal exposure. A low formation rate of lyso derivatives during storage, that is, excellent storage stability, which is available from the foregoing possibility, will be demonstrated in Test 1 to be described subsequently herein.

In addition, a liposome suspension and a drug solution can be mixed at a low temperature (around 25°C) where the incorporation of the drug hardly takes place, and can then be heated immediately to a high temperature at which a high loading efficiency is available. It is, therefore, considered that the drug can be evenly incorporated in the liposomes.

From the above-described results, it has been found that according to the present invention, a liposome preparation with a drug (Dox) loaded sufficiently from the standpoint of clinical effects can be obtained in an extremely short overall heating time (approx. 3 to 10 seconds) provided that the temperature of a sample at the outlet of the heat exchanger is 45°C or higher.

(Comparative Examples 1 to 3)

A Dox-loaded liposome preparation was obtained by conducting likewise the steps of Preparation Example 1 except that the total volume (15 L) of the liposome suspension and drug suspension was subjected to the conventional heated stirring/mixing step instead of the drug incorporation step (4) of Preparation Example 1. In the heated stirring/mixing, the liposome suspension (9.8 L) subjected to an external medium exchange in the external medium exchange step (3) was placed in a 20-L tank and was heated beforehand from room temperature to 60°C over 20 minutes (in which the heating at 40°C and higher lasted for 10 minutes). To the thus-heated liposome suspension, a predetermined amount of the same Dox solution (10 mg/mL) as in the above-described preparation example was added after heating it to 60°C beforehand. After mixing them together, stirring was conducted at 60°C for 60 minutes (Comparative Example 1), 10 seconds (Comparative Example 2) or 20 seconds (Comparative Example 3) to incorporate the drug. The percent drug incorporations of the thus-obtained Dox-loaded liposome preparations are shown in Table 2.

[Table 2]

| | Temperature(°C) | Time (s) | Percent drug incorporation (%) |
|---|---|---|---|
| Comparative Example 1 | 60 | 3600 | 98.1 |
| Comparative Example 2 | 60 | 10 | 90.2 |
| Comparative Example 3 | 60 | 20 | 93.4 |

In the heated stirring/mixing conducted in the comparative examples, values higher than 90% were achieved as percent drug incorporations in the respective examples. At the experiment scale of the comparative examples, time of one minute or longer is needed to mix a liposome suspension and a drug solution together so that the liposome suspension and drug solution are in contact with each other in the preheated states, respectively. Presumably, the drug is hence incorporated sequentially in the order of its contact with the liposome suspension, and as a result, the heating time in the drug incorporation step becomes 10 seconds, 20 seconds or longer to provide a sufficient percent incorporation. In other words, the above-described percent incorporation is achieved by spending 10 minutes or longer as an overall heating time including the preheating. Further, this percent incorporation is an average value for all the liposome particles. As the drug is added in a significantly small amount in this experiment system compared with the amount of the drug which can be incorporated in the liposomes in the mixed liposome suspension, the drug is presumably incorporated in some liposome particles before the drug comes into contact with all the liposome particles so that variations may occur in percent drug incorporation among the liposome particles.

(Test 1) Storage stability of Dox-loaded liposome preparation

The individual Dox-loaded liposome preparations formed in Preparation Example 2 and Comparative Example 1 were heated at 40°C for a predetermined time. From the heated Dox-loaded liposome preparations, samples were collected every week, and the percent formation of lipid degradation products (lyso derivatives) were determined by the high performance liquid chromatograph. The results are shown in Table 3 and FIG. 5. In addition, the measurement results of particle sizes of the liposome preparations at the respective time points are also shown in Table 3.

Measurement method of lipid degradation products (lyso derivatives): high performance liquid chromatography.

The particle sizes are average particle sizes measured by a dynamic light scattering particle analyzer ("Zetasizer 3000," Malvern Instruments).

[Table 3]

| Incorporation conditions / Storage period (W) at 40°C | Percent lipid degradation (%) | | | | Particle size (nm) | | | |
|---|---|---|---|---|---|---|---|---|
| | Preparation Example 2 | Comp. Ex. 1 | Comp. Ex. 2 | Comp. Ex. 3 | Preparation Example 2 | Comp. Ex. 1 | Comp. Ex. 2 | Comp. Ex. 2 |
| 0 | 0.5 | 0.2 | 0.3 | 0.4 | 90.3 | 90.4 | 90.5 | 90.3 |
| 1 | 7.7 | 12.7 | 11.5 | 11.7 | 90.2 | 90.0 | 90.3 | 90.2 |
| 2 | 12.7 | 18.0 | 17.6 | 17.2 | 91.4 | 90.6 | 90.3 | 90.4 |

It has been demonstrated that the percent formation of lipid degradation products can be suppressed by using the method of the present invention. Described specifically, the method of the present invention can raise the temperature of a mixture, which contains liposomes and a drug, in an extremely short time, and therefore, the incorporation of the drug can be completed during feeding through a heat exchanger. Compared with the conventional method that requires to conduct preheating for the entirety of prepared empty liposomes before the loading of the drug, substantial shortening of thermal exposure was feasible. This shortening presumably led to improvements in the stability of the lipids, and hence, to a reduction in the percent formation of lipid degradation products. Further, no variations were observed in particle size during the storage period at 40°C, and excellent physiochemical stability is suggested on the preparation.

From the above results, the method according to the present invention has been found that it can incorporate a drug in an extremely short time while assuring physiochemical stability, it can also improve the stability of lipid(s), and from the standpoint of production time, the standpoint of quality such as the uniformity in the amount of incorporated drug and the standpoint of the stability of the preparation, it is fully satisfactory.

## Claims

1. A method for producing a liposome preparation by using a remote loading method, which comprises a drug incorporation step that heats a mixture of a preliminarily prepared suspension of liposomes and a drug by rapid heating means to a temperature from not lower than a phase transition point of membranes of said liposomes to not higher than 80°C to incorporate said drug into said liposomes,
wherein said rapid heating means is a heat exchanger interposed in a feed pipe.

2. The method according to claim 1, wherein said drug is incorporated into said liposomes by an ion gradient method.

3. The method according to claim 1 or 2, wherein a heat-exchanger residence time in said heat exchanger is from 3 to 120 seconds.

4. The method according to any one of claims 1 to 3, which comprises performing an elimination step for any unloaded drug after said drug incorporation step.

5. The method according to any one of claims 1 to 4, further comprising a temperature down step comprising cooling the mixture to not higher than the phase transition point of membranes of said liposomes after the heating.

## Patentansprüche

1. Verfahren zum Produzieren einer Liposomzubereitung unter Verwendung eines Remote-Ladeverfahrens, welches einen Arzneimittelinkorporationsschritt umfasst, der eine Mischung einer zuvor angefertigten Suspension von Liposomen und eines Arzneimittels mittels Schnellheizeinrichtung auf eine Temperatur von nicht weniger als ein Phasenübergangspunkt von Membranen der Liposome bis nicht höher als 80°C erhitzt, um das Arzneimittel in die Liposome zu inkorporieren,
wobei die Schnellheitseinrichtung ein in einer Zuführleitung eingefügter Wärmetauscher ist.

2. Verfahren nach Anspruch 1, wobei das Arzneimittel in die Liposome durch ein Ionengradientenverfahren inkorporiert wird.

3. Verfahren nach Anspruch 1 oder 2, wobei eine Wärmetauscherverweilzeit in dem Wärmetauscher von 3 bis 120 Sekunden beträgt.

4. Verfahren nach einem der Ansprüche 1 bis 3, welches das Durchführen eines Eliminationsschritts für jegliches nicht geladene Arzneimittel nach dem Arzneimittelinkorporationsschritt umfasst.

5. Verfahren nach einem der Ansprüche 1 bis 4, das ferner einen Temperaturerniedrigungsschritt umfasst, der das Abkühlen der Mischung auf nicht höher als den Phasenübergangspunkt von Membranen der Liposome nach dem Erwärmen umfasst.

## Revendications

1. Procédé de production d'une préparation de liposomes en utilisant un procédé de chargement à distance, qui comprend une étape d'incorporation de médicament qui consiste à chauffer un mélange d'une suspension préalablement préparée de liposomes et d'un médicament par un moyen de chauffage rapide à une température comprise entre une température qui n'est pas inférieure à un point de transition de phase de membranes desdits liposomes et une température qui n'est pas supérieure à 80°C pour incorporer ledit médicament dans lesdits liposomes,
dans lequel ledit moyen de chauffage rapide est un échangeur de chaleur interposé dans un tuyau d'alimentation.

2. Procédé selon la revendication 1, dans lequel ledit médicament est incorporé dans lesdits liposomes par un procédé à gradient ionique.

3. Procédé selon la revendication 1 ou 2, dans lequel un temps de séjour dans un échangeur de chaleur dans ledit échangeur de chaleur est compris entre 3 et 120 secondes.

4. Procédé selon l'une quelconque des revendications 1 à 3, qui comprend le fait d'exécuter une étape d'élimination de tout médicament non chargé après ladite étape d'incorporation de médicament.

5. Procédé selon l'une quelconque des revendications 1 à 4, comprenant en outre une étape d'abaissement de température comprenant le refroidissement du mélange à une température qui n'est pas supérieure au point de transition de phase de membranes desdits liposomes après le chauffage.

# FIG.1

3
1
CONSTANT-TEMPERATURE HEATING BATH
HEAT EXCHANGER
PUMP
2
4
5
GLASS BOTTLE (MIXTURE OF LIPOSOME SUSPENSION AND DRUG SOLUTION)
GLASS BOTTLE (DRUG-LOADED LIPOSOME SUSPENSION)

# FIG.2

K (mg/mL/min)
1.4
1.2
1.0
0.8
0.6
0.4
0.2
0.0
20
25
30
35
40
45
50
55
TEMPERATURE (°C)

FIG.3

PERCENT DRUG INCORPORATION (%)
100
80
60
40
20
0
0
5
10
15
20
TIME (min)
FIG.4
PERCENT DRUG INCORPORATION (%)
100
80
60
40
20
0
35
40
45
50
OUTLET TEMPERATURE (°C)

FIG.5

PERCENT FORMATION OF LYSO DERIVATIVES (%)
20
15
10
5
0
COMPARATIVE EXAMPLE 1
PRESENT INVENTION
0
1
2
STORAGE PERIOD (W)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2659136 B
- WO 2005092388 A
- US 6355268 B1
- US 5939096 A
- EP 0800822 A2
- EP 1190705 A1
- US 2004071768 A1
- WO 8601102 A1
- US 5393530 A
- JP SHO61161246 B
- JP TH5508626 B
- JP HEI2292246 B
- JP HEI4108391 B
- WO 9742166 A
- JP 2766691 B
- JP TH07112968 B
- US 5192549 A


### Non-patent literature cited in the description


- **TERADA, YOSHIMURA et al.** Liposomes in Life Science'' (in Japanese). Springer-Verlag Tokyo, 1992
- **G. GREGORIADIS.** Liposome Technology Liposome Preparation and Related Techniques. CRC Press, vol. I-III